# EUROPEAN PATENT SPECIFICATION

(11) **EP 1 462 109 B1**
(45) Date of publication and mention of the grant of the patent: **13.02.2008**
(21) Application number: 03425195.9
(22) Date of filing: 28.03.2003
(51) Int. Cl.: A61K 35/12, A61K 38/39, A61K 9/70, A61K 9/06, A61L 15/34, A61L 15/50, A61P 7/04, A61P 17/00

(54) **Hemostatic, emollient and lubricating preparations containing pork fat extract (lard)**
Schweineschmalz enthaltende Zusammensetzungen als Hämostatikum mit Gleitmittel- und Hautrückfettender Wirkung
Préparations hémostatiques, émollientes et lubrifiantes contenant du saindoux

(43) Date of publication of application: 29.09.2004
(73) Proprietor: D.M.G. Italia Srl, 00040 Pomezia (RM) (IT)
(72) Inventor: Mercuri, Luigi, 00181 Roma (IT)
(74) Representative: Sarpi, Maurizio

(56) References cited:
- EP-A- 0 923 935
- CH-A- 676 324
- US-A- 147 898
- US-A- 3 279 993
- US-A- 4 578 067

## Description

The present invention concerns the field of medical devices, and more particularly a composition containing natural extract of pork fat having strong haemostatic, emollient and lubricant activity.

Aim of the present invention is to provide a composition able to effectively and rapidly block bleeding after various haemorrhagic events, i.e. sporadic haemorrhages in healthy subjects or haemorrhages due to pathologies of the hematic and coagulation systems. At the same time, said composition, showing lubricating and emollient activity, induces tissue regeneration and avoids the formation of solid clots and inhibits imperfect wound healing. A specific characteristic of the present invention, being exclusively of natural origin, is that, it is safely usable and well tolerated and it does not induce local or systemic allergic reactions. Moreover, the object of the present invention, can be industrially prepared in an extremely economic way because it is simply an extract of animal fat enriched in collagen, calcium and phospholipids.

Up to now, there various preparations with haemostatic activity, have been proposed, which are useful in medical and surgical procedures and for treatment of wounds, wherein haemostatic /procoagulation factors such as phospholipids, calcium, and collagen are present.

For instance, collagen fibers are present in form of partial acid salt in US Patent 4,578,067, while aqueous dispersion of lipid-thromboplastin is disclosed in US Patent 3,279,993, and haemostatic phospholipids are disclosed in CH 676324.

Also, it is known from EP 0 923 935 a locally administrable preparation for treating affected tissues which contains tannic acid and potassium aluminum sulfate.

From the US Patent 147,898 it is known an empiric ointment for piles made by melting hog's lard with hog's hair. However no whatsoever evidence has been produced in order to claim an haemostatic effect of said ointment or of hog's lard per se.

It is well known that the haemostatic process involves three consecutive steps:
1. the vascular-platelet phase;
2. the hemocoagulative phase;
3. fibrinolytic phase.

During the first phase, platelets play a key role in the activation of the coagulative process. Platelets adhere to vessel walls, form white thrombus and induce the first tamponade of bleeding. In fact, the interaction between platelets and endothelium collagen induce their activation and the release, on their surface, of pro-coagulative phospholipidic molecules, whose function is to activate the coagulative cascade which lead to the formation of the fibrin clot.

In view of these physiological considerations, many studies are nowadays focused on lipidic molecules involved in the haemostatic process to evaluate their therapeutic application.

In vivo and in vitro experiments were conducted by the Applicant to evaluate the haemostatic activity of pork fat extract (Type 1) and its enriched formulation (type 2) enriched wherein with calcium, collagen and vegetal phospholipids containing up to 90 % of phosphatidylcholine (as commercially available under the name of Emulmetik 950, LUCAS MEYER GmbH, Hamburg, Germany) are added.

In addition, to obtain an industrially manufactured product glyceryl stearate, methyl parahydroxybenzoate and propyl parahydroxybenzoate were added.

In particular, glyceryl stearate is a lipidic factor of consistence which maintain the composition in the solid phase at high temperatures. The two protective agents methyl parahydroxybenzoate and propyl parahydroxybenzoate, protect the composition from microbial colonization.

The enriched formulation, called Type 2, has the following composition (for 100 grams of product):
pork fat extract (lard) 85 - 90 gr
BHT (buthylhydroxytoluen) 0,01 - 0,2 gr
Vitamin E acetate 0,1 - 1,0 gr
soy oil 0,5 - 5,0 gr
Hydrolysed collagen 0,1 - 1,0 gr
phospholipidic extract (PC 90%) 0,1 - 1,0 gr
KCl 0,1 - 1,0 gr
MgCl 0,1 - 1,0 gr
CaCl 0,1 - 1,0 gr
Glyceryl stearate 1,0 - 8,0 gr
methyl parahydroxybenzoate 0,01 - 0,5 gr
propyl parahydroxybenzoate of 0,01 - 0,5 gr

In vitro experiments were conducted to evaluate the stimulating activity of said fat extract on coagulation in human blood and/or plasma samples.

### COAGULATION TIME TEST

The coagulation time (TC) was measured on whole blood by visual inspection of the clot according to the well-known protocol called Lee-White Modified Test ("Human blood coagulation, haemostasis and thrombosis" Edited by Rosemary Biggs, 1972, Oxford, Blakwell Scientific Publications). This test was conducted on 12 whole blood samples of 6 ml, (drawn from subjects showing an apparently normal coagulative system), distributed in aliquots of 1 ml in 6 glass-made test tubes and exposed to a temperature of 37°C (normal human body temperature). Three test tubes were used as controls to evaluate spontaneous TC, and in the remaining 3 test tubes 100 µl of purified pork fat extract (Type 1) were added. The obtained results are shown in the following table 1.

**TABLE 1**

| SAMPLES | TC (min.) control | | | TC (min.) Type 1 | | |
|---|---|---|---|---|---|---|
| | **1** | **2** | **3** | **1** | **2** | **3** |
| I | 6,4 | 8,5 | 8 | 4,2 | 4 | 6,5 |
| II | 7,3 | 6,4 | 7,2 | 5,1 | 4,2 | 5 |
| III | 7 | 6,3 | 8 | 3,1 | 3,5 | 5,2 |
| IV | 10 | 9 | 6,4 | 5 | 4,3 | 5 |
| V | 6 | 6,3 | 6,1 | 6,5 | 5,5 | 4,2 |
| VI | 11 | 9,2 | 6 | 6 | 5,5 | 3,2 |
| VII | 7 | 7,4 | 4,5 | 6,5 | 6 | 5 |
| VIII | 6 | 5,4 | 5,2 | 3,2 | 3 | 4,4 |
| IX | 4,3 | 7,2 | 6 | 4,2 | 4,4 | 3 |
| X | 5 | 8 | 9,2 | 5,1 | 4 | 5,5 |
| XI | 3 | 6,3 | 5 | 3,4 | 5 | 6,1 |
| XII | 7 | 5,5 | 5 | 8 | 6,2 | 6 |

Data reported in table 1 show that coagulation time (TC) in samples added with Type 1 is significantly reduced in comparison to controls.

### PARTIAL TROMBOPLASTINE TIME TEST

Coagulation time was evaluated measuring another typical parameter: the Partial Tromboplastine Time (PTT). The commercially available diagnostic kit Thorombofax (Ortho Diagnostic System Inc. USA) was used by replacing the phospholipidic emulsion, given with the commercially available diagnostic kit, with 200 µl of Type 1. The test was conducted adding to 0,2 ml of human plasma 0,2 ml of phospholipidic emulsion of the diagnostic kit Thorombofax in a first test-tube and 0,2 ml of Type 1 in the second test tube, and incubating at 37°C (human body temperature). The time was manually measured with a chronometer, considering as time t=0 (minutes) the addition of 0,2 ml calcium chloride (0,025 M) in the test tube; PTT was recorded as the time between calcium addition to the appearance of fibrin fibers. Obtained results are shown in the following Table 2.

**TABLE 2**

| SAMPLES | PTT (sec.) control | PTT (sec.) TYPE 1 |
|---|---|---|
| I | 28 | 34 |
| II | 32 | 33 |
| III | 26 | 28 |
| IV | 28 | 36 |
| V | 33 | 40 |
| VI | 35 | 38 |
| VII | 33 | 36 |
| VIII | 27 | 32 |
| IX | 30 | 30 |
| X | 25 | 29 |

The results in table 2 show that PTT values are similar in all the samples and the effectiveness of the claimed composition is comparable with other commercially available phospholipidic preparations.

### PARTIAL THROMBOPLASTIN TIME TEST ON THE ENRICHED FORMULATION

Partial thromboplastin time was evaluated on the Type 2 formulation, enriched with collagen, calcium and phospholipids and added with glyceryl stearate, methyl parahydroxybenzoate and propyl parahydroxybenzoate, having the following composition:
pork fat extract (lard) 89,14 gr
BHT (butylhydrossitoluen) 0,1 gr
Vitamin E acetate 0,5 gr
soy oil 2 gr
Hydrolysed collagen 0,5 gr
phospholipidic extract (PC 90%) 0,2 gr
KC1 0,5 gr
MgCl 0,5 gr
CaCl 0,5 gr
Glyceryl stearate 6 gr
methyl para-hydroxybenzoate 0,05 gr
propyl para-hydroxybenzoate of 0,01 gr
14 plasma samples, drawn from subjects showing an apparently normal coagulative system, were prepared. PTT was measured in presence of phospholipidic emulsion (commercial kit Thorombofax Ortho Diagnostic System Inc. USA) or Type 1 or or Type 2.

In three different test tubes containing 0,2 ml of plasma the following substance: 0,2 ml of Thorombofax phospholipidic emulsion, 0,2 ml of Type 1 and 0,2 ml of Type 2 were added. After 15 minutes incubation at 37°C (human body temperature), 0,2 ml of calcium was added starting the chronometer, as previously described. Obtained results are shown in the following table 3.

**TABLE 3**

| Samples | PTT (sec.) control | PTT (sec.) TYPE 1 | PTT (sec.) TYPE 2 |
|---|---|---|---|
| I | 30 | 36 | 32 |
| II | 29 | 34 | 34 |
| III | 28 | 37 | 30 |
| IV | **28** | 37 | **22** |
| V | 30 | 33 | 33 |
| VI | 29 | 39 | 35 |
| VII | **26** | 33 | **20** |
| VIII | 27 | 37 | 30 |
| IX | 19 | 30 | 31 |
| X | 22 | 39 | 29 |
| XI | **24** | 36 | **14** |
| XII | 18 | 35 | 23 |
| XIII | **21** | 31 | **18** |
| XIV | 26 | 29 | 29 |

The results of table 3 underline that plasma coagulation occurs in comparable times, but in four samples of plasma added with type 2 (marked in bold character) formation of fibrin was more rapid.

In conclusion, in vitro experimental tests show that pork fat extract (Type 1) and especially its enriched formulation (Type 2) added with procoagulation factors as collagen, calcium and phospholipids, both objects of the invention, have a strong coagulative activity comparable to other phospholipid emulsions, commonly used in laboratory diagnostic.

### IN VIVO EXPERIMENTS

In vivo clinical experiments were conducted (in collaboration with the Department of Hematology, S. Giovanni Addolorata Hospital, Rome) to evaluate the therapeutic effects of the pork fat extract enriched formulation (type 2) added with procoagulating factors, in the treatment cutaneous-mucous hemorragic events.

In the first phase, 30 eligible patients were selected among those ordinarily hospitalised to have a statistic sample having the following characteristics:
- Patients with cutaneous or mucous hemorrhages from "light" to "moderate" due to diagnostic-therapeutic operations i.e. osteo-medulla biopsy or dental extraction;
- Patients with mucous hemorrhages, particularly epistaxis, spontaneous or due to vascular defects (nose varix) or hematological defects as plastocytopenia or various coagulopathy.

The pork fat preparation enriched with procoagulating factors, object of the invention, in the previously described formulation (Type 2), was stratified on sterile gauze with iodine 10%. Said gauzes were prepared in bands of 2cm x 1m to be easily suited on the hemorragic focus; in case of tamponade of small cutaneous hemorrhages after biopsy or dental extraction the bands were prepared in panels of reduced dimensions (2 cm x 2 cm), for the so called "plate" medications or pack; in addition, tampons were prepared for the treatment of nasal hemorrhages.

The activity of the claimed preparation was evaluated measuring the time necessary to block bleeding, considering that the median plugging time is of about 4-5 days using traditional hemostatic products. Obtained results are shown in the following table 4.

**TABLE 4**

| | | | | |
|---|---|---|---|---|
| | **Diagnosis** | **Hemorrhage** | **Treatment** | **Plugging Time** |
| 1 | plastocytopenia | epistaxis | tamponage and infusion of concentrate platelets | 4 days |
| 2 | nose varix | epistaxis | tamponage | 3 days |
| 3 | plastocytopenia | epistaxis | tamponage | 5 days |
| 4 | plastocytopenia | epistaxis | tamponage | 5 days |
| 5 | bone biopsy | cutaneous hemorrhage | medication | 2 hours |
| 6 | acquired coagulopathy (cirrhosis) | epistaxis | tamponage | 5 days |
| 7 | plastocytopenia | epistaxis | tamponage and infusion of concentrate platelets | 5 days |
| 8 | epulis ablation | gingival hemorrhage | tamponage | 4 days |
| 9 | plastocytopenia | epistaxis | tamponage | 4 days |
| 10 | nose varix | epistaxis | tamponage | 3 days |
| 11 | molar sup. extraction. | gingival hemorrhage | pack | 2 days |
| 12 | plastocytopenia | epistaxis | tamponage and infusion of concentrate platelets | 5 days |
| 13 | dehiscence of suture with bleeding | cutaneous hemorrhage | medication | 8 days |
| 14 | plastocytopenia and nasal infection | epistaxis | medication and antibiotics treatment | 5 days |
| 15 | nose varix plastocytopenia | epistaxis | tamponage | 4 days |
| 16 | bone biopsy | cutaneous hemorrage | medication | 4 days |
| 17 | plastocytopenia | gingival hemorrhage | tamponage | 5 days |
| 18 | bone biopsy | cutaneous hemorrhage | medication | 7 days |
| 19 | nose varix | epistaxis | tamponage | 5 days |
| 20 | plastocytopenia | epistaxis | tamponage | 6 days |
| 21 | bone biopsy | cutaneous hemorrage | medication | 5 days |
| 22 | plastocytopenia | epistaxis | tamponage | 5 days |
| 23 | acquired coagulopathy post-hepatic cirrhosis) | epistaxis | tamponage | 5 days |
| 24 | bone biopsy | cutaneous hemorrhage | medication | 4 days |
| 25 | bone biopsy | cutaneous hemorrhage | medication | 2 days |
| 26 | molar inf. extraction. | gingival hemorrhage | pack | 6 days |
| 27 | plastocytopenia | epistaxis | tamponage and infusion of concentrate platelets | 7 days |
| 28 | acquired coagulopathy | epistaxis | tamponage | 5 days |
| 29 | plastocytopenia | epistaxis | tamponage | 5 days |
| 30 | bone biopsy | cutaneous hemorrhage | tamponage | 6 days |

Clinical experiments demonstrate that the preparation object of the invention, have good direct haemostatic activity in suitable times.

In particular, the statistic elaboration of data highlights what hereinafter summarised:
- the median plugging time in 18 cases of epistaxis was of 4,7 days;
- the median plugging time in 5 cases of gingival hemorrhage was of 4,2 days;
- the median plugging time in 7 cases of cutaneous hemorrhages due to various causes was of 5,2 days.

In all cases, the median plugging time was reduced in comparison to traditional local haemostatic means.

Moreover, local or systemic intolerance to the claimed preparation was never observed.

It has to be underlined that, patients of the statistic sample used in the experimental protocol, were all under high haemorragic risk being some of them affected by evolutionary haemorragic pathologies, where local haemorragic events are more frequent and are the expression of a wider damage of the hemo-coagulative system

In conclusion, it is likely to assume that the haemostatic activity of the preparation, object of the present invention, is greater on "healthy" subjects with sporadic and/or lacalised haemorragies.

## Claims

1. Use of purified pork fat extract (lard) for the preparation of a topical composition for use as a haemostatic, emollient and lubricant.

2. Use of purified pork fat extract (lard) enriched with collagen, calcium and phospholipids for the preparation of a topical composition for use as a haemostatic, emollient and lubricant.

3. Topical haemostatic preparation having the following composition (total weight 100 gr):
pork fat extract (lard) 85 - 90 gr
BHT (butylhydroxytoluen) 0,01 - 0,2 gr
Vitamin E acetate 0,1 - 1,0 gr
soy oil 0,5 - 5,0 gr
Hydrolysed Collagen 0,1 - 1,0 gr
phospholipidic extract (PC 90%) 0,1 - 1,0 gr
KCl 0,1 - 1,0 gr
MgCl 0,1 - 1,0 gr
CaCl 0,1 - 1,0 gr

4. Topical haemostatic preparation having the composition (total weight 100gr) according to claim 3 wherein glyceryl stearate 1,0 - 8,0 gr as consistence factor, methyl para-hydroxybenzoate 0,01 - 0,5 gr and propyl para-hydroxybenzoate 0,01 - 0,5 as preserving agents, are added.

5. Topical haemostatic preparation having the following composition (total weight 100 gr):
pork fat extract (lard) 89,14 gr
BHT (butylhydroxytoluen) 0,1 gr
Vitamin E acetate 0,5 gr
soy oil 2 gr
Hydrolysed Collagen 0,5 gr
phospholipidic extract (PC 90%) 0,2 gr
KCl 0,5 gr
MgCl 0,5 gr
CaCl 0,5 gr
Glyceryl stearate 6 gr
methyl para-hydroxybenzoate 0,05 gr
propyl para-hydroxybenzoate of 0,01 gr

6. Topical haemostatic preparation having the composition (total weight 100gr) according to claim 5 wherein Glyceryl stearate 6,0 gr as consistence factor, methyl parahydroxybenzoate 0,01 - 0,5 gr and propyl parahydroxybenzoate 0,01 - 0,5 as preserving agents, are added.

7. Use of purified pork fat extract (lard) enriched with collagen, calcium and phospholipids for the preparation of a haemostatic medication having the form of gauzes or bandages.

8. Use of purified pork fat extract (lard) enriched with collagen, calcium, phospholipids, glyceryl stearate, methyl parahydroxybenzoate and propyl parahydroxybenzoate for the preparation of a haemostatic medication having the form of gauzes or bandages

9. Haemostatic medication **characterised in** being in the form of gauzes or bandages imbibited or soaked with the preparation of claim 5.

10. Haemostatic medication **characterised in** being in the form of gauzes or bandages imbibited or soaked with the preparation of claim 6.

11. Use of purified pork fat extract (lard) enriched with collagen, calcium and phospholipids for the preparation of a haemostatic medication having the form of pomade or ointment.

12. Use of purified pork fat extract (lard) enriched with collagen, calcium, phospholipids, glyceryl stearate, methyl parahydroxybenzoate and propyl parahydroxybenzoate for the preparation of a haemostatic medication having the form of pomade or ointment.

13. Haemostatic medication **characterised in** being in the form of pomade or ointment containing, as active principle, the composition of claim 5.

14. Haemostatic medication **characterised in** being in the form of pomade or ointment containing, as active principle, the composition of claim 6.

## Patentansprüche

1. Verwendung eines gereinigten Schweinefettextrakts (Schweineschmalz) zur Herstellung einer topischen Zusammensetzung zur Verwendung als Haemostatikum, Weichmacher und Gleitmittel.

2. Verwendung eines gereinigten Schweinefettextrakts (Schweineschmalz), angereichert mit Collagen, Kalzium und Phospholipiden, zur Herstellung einer topischen Zusammensetzung zur Verwendung als Haemostatikum, Gleitmittel und Weichmacher.

3. Topische haemostatische Zubereitung mit der nachfolgenden Zusammensetzung (Gesamtgewicht 100 g):
Schweinefettextrakt (Schweineschmalz) 85 - 90 g
BHT (Butylhydroxytoluol) 0,01 - 0,2 g
Vitamin E-acetat 0,1 - 1,0 g
Sojaöl 0,5 - 5,0 g
Hydrolysiertes Collagen 0,1- 1,0 g
Phospholipidextrakt (PC 90%) 0,1 - 1,0 g
KCl 0,1 - 1,0 g
MgCl 0,1 - 1,0 g
CaCl 0,1 - 1,0 g.

4. Topische haemostatische Zubereitung mit der Zusammensetzung (Gesamtgewicht 100 g) nach Anspruch 3, wobei Glycerinstearat 1,0 - 8,0 g als Konsistenzfaktor, Methyl-para-hydroxybenzoat 0,01 - 0,5 g und Propyl-para-hydroxybenzoat 0,01 - 0,5 als Konservierungsmittel zugesetzt werden.

5. Topische haemostatische Zubereitung mit der nachfolgenden Zusammensetzung (Gesamtgewicht 100 g):
Schweinefettextrakt (Schweineschmalz) 89,14 g
BHT (Butylhydroxytoluol) 0,1 g
Vitamin E-acetat 0,5 g
Sojaöl 2 g
Hydrolysiertes Collagen 0,5 g
Phospholipidextrakt (PC 90%) 0,2 g
KCl 0,5 g
MgCl 0,5 g
CaCl 0,5 g
Methyl-para-hydroxybenzoat 0,05 g
Propyl-para-hydroxybenzoat 0,01 g.

6. Topische haemostatische Zubereitung mit der Zusammensetzung (Gesamtgewicht 100 g) nach Anspruch 5, wobei Glycerinstearat 6,0 g als Konsistenzfaktor, Methyl-para-hydroxybenzoat 0,01 - 0,5 g und Propyl-para-hydroxybenzoat 0,01 - 0,5 als Konservierungsmittel zugesetzt werden.

7. Verwendung eines gereinigten Schweinefettextrakts (Schweineschmalz), angereichert mit Collagen, Kalzium und Phospholipiden, zur Herstellung einer haemostatischen Medikation in Form von Gaze (Mull) oder Bandagen.

8. Verwendung eines gereinigten Schweinefettextrakts (Schweineschmalz), angereichert mit Collagen, Kalzium, Phospholipiden, Glycerinstearat, Methyl-para-hydroxybenzoat und Propyl-para-hydroxybenzoat, zur Herstellung einer haemostatischen Medikation in Form von Gaze (Mull) oder Bandagen.

9. Haemostatische Medikation, **dadurch gekennzeichnet, dass** sie in Form von Gaze oder Bandagen vorliegt, getränkt oder absorbiert mit der Zubereitung nach Anspruch 5.

10. Haemostatische Medikation, **dadurch gekennzeichnet,**
**dass** sie in Form von Gaze oder Bandagen vorliegt, getränkt oder absorbiert mit der Zubereitung nach Anspruch 6.

11. Verwendung von gereinigtem Schweinefettextrakt (Schweineschmalz), angereichert mit Collagen, Kalzium und Phospholipiden, zur Herstellung einer haemostatischen Medikation in Form einer Pomade oder Salbe.

12. Verwendung von gereinigtem Schweinefettextrakt (Schweineschmalz), angereichert mit Collagen, Kalzium und Phospholipiden, Glycerinstearat, Methyl-para-hydroxybenzoat und Propyl-para-hydroxybenzoat, zur Herstellung einer haemostatischen Medikation in Form einer Pomade oder Salbe.

13. Haemostatische Medikation, **dadurch gekennzeichnet,**
**dass** sie in Form einer Pomade oder Salbe vorliegt, enthaltend, als aktives Prinzip, die Zusammensetzung nach Anspruch 5.

14. Haemostatische Medikation, **dadurch gekennzeichnet,**
**dass** sie in Form einer Pomade oder Salbe vorliegt, enthaltend, als aktives Prinzip, die Zusammensetzung nach Anspruch 6.

## Revendications

1. Utilisation d'extrait de gras de porc purifié (saindoux) pour la préparation d'une composition topique prévue pour être utilisée comme hémostatique, émollient et lubrifiant.

2. Utilisation d'extrait de gras de porc purifié (saindoux) enrichi avec du collagène, du calcium et des phospholipides pour la préparation d'une composition topique prévue pour être utilisée comme hémostatique, émollient et lubrifiant.

3. Préparation hémostatique topique présentant la composition suivante (poids total de 100 g) :
. extrait de gras de porc (saindoux) 85 à 90 g
. BHT (butylhydroxytoluène) 0,01 à 0,2 g
. acétate de vitamine E 0,1 à 1,0 g
. huile de soja 0,5 à 5,0 g
. collagène hydrolisé 0,1 à 1,0 g
. extrait phospholipidique (PC 90%) 0,1 à 1,0 g
. KCl 0,1 à 1,0 g
. MgCl 0,1 à 1,0 g
. CaCl 0,1 à 1,0 g

4. Préparation hémostatique topique présentant la composition selon la revendication 3 (poids total de 100 g), dans laquelle on ajoute de 1,0 à 8,0 g de stéarate de glycéryle comme agent de consistance, de 0,01 à 0,5 g de parahydroxybenzoate de méthyle et de 0,01 à 0,5 g de parahydroxybenzoate de propyle comme agents conservateurs.

5. Préparation hémostatique topique présentant la composition suivante (poids total de 100 g) :
. extrait de gras de porc (saindoux) 89,14 g
. BHT (butylhydroxytoluène) 0,1 g
. acétate de vitamine E 0,5 g
. huile de soja 2,0 g
. collagène hydrolisé 0,5 g
. extrait phospholipidique (PC 90%) 0,2 g
. KC1 0,5 g
. MgCl 0,5 g
. CaCl 0,5 g
. stéarate de glycéryle 6,0 g
. parahydroxybenzoate de méthyle 0,05 g
. parahydroxybenzoate de propyle 0,01 g

6. Préparation hémostatique topique présentant la composition selon la revendication 5 (poids total de 100 g), dans laquelle on ajoute 6,0 g de stéarate de glycéryle comme agent de consistance, de 0,01 à 0,5 g de parahydroxybenzoate de méthyle et de 0,01 à 0,5 g de parahydroxybenzoate de propyle comme agents conservateurs.

7. Utilisation d'extrait de gras de porc purifié (saindoux) enrichi avec du collagène, du calcium et des phospholipides pour la préparation d'un médicament hémostatique sous la forme de gazes ou de bandages.

8. Utilisation d'extrait de gras de porc purifié (saindoux) enrichi avec du collagène, du calcium, des phospholipides, du stéarate de glycéryle, du parahydroxybenzoate de méthyle et du parahydroxybenzoate de propyle pour la préparation d'un médicament hémostatique sous la forme de gazes ou de bandages

9. Médicament hémostatique **caractérisé en ce qu'**il se présente sous la forme de gazes ou de bandages imprégnés ou imbibés de la préparation selon la revendication 5.

10. Médicament hémostatique **caractérisé en ce qu'**il se présente sous la forme de gazes ou de bandages imprégnés ou imbibés de la préparation selon la revendication 6.

11. Utilisation d'extrait de gras de porc purifié (saindoux) enrichi avec du collagène, du calcium et des phospholipides pour la préparation d'un médicament hémostatique sous la forme d'une pommade ou d'un onguent.

12. Utilisation d'extrait de gras de porc purifié (saindoux) enrichi avec du collagène, du calcium, des phospholipides, du stéarate de glycéryle, du parahydroxybenzoate de méthyle et du parahydroxybenzoate de propyle pour la préparation d'un médicament hémostatique sous la forme d'une pommade ou d'un onguent.

13. Médicament hémostatique **caractérisé en ce qu'**il se présente sous la forme d'une pommade ou d'un onguent contenant, en tant que principe actif, la composition selon la revendication 5.

14. Médicament hémostatique **caractérisé en ce qu'**il se présente sous la forme d'une pommade ou d'un onguent contenant, en tant que principe actif, la composition selon la revendication 6.
